# EUROPEAN PATENT SPECIFICATION

(11) **EP 1 299 537 B1**
(45) Date of publication and mention of the grant of the patent: **20.12.2006**
(21) Application number: 01947636.5
(22) Date of filing: 06.07.2001
(51) Int. Cl.: C12N 15/12, C07K 14/705, C07C 323/41, C07C 323/59, A61K 47/48

(54) **COMPOUNDS TARGETED TO CELLULAR LOCATIONS**
AUF ZELLULÄRE ZIELE GERICHTETE VERBINDUNGEN
COMPOSES CIBLES SUR DES POSITIONS CELLULAIRES

(30) Priority: 07.07.2000 GB 0016811
(43) Date of publication of application: 09.04.2003
(73) Proprietor: AdProTech Limited, Saffron Walden, Essex CB10 1XL (GB)
(72) Inventor: ROWLING, Pamela Jane Elizabeth, AdProTech Limited, Saffron Walden, Essex CB10 1XL (GB); SMITH, Geoffrey Paul, AdProTech Limited, Saffron Walden, Essex CB10 1XL (GB); RIDLEY, Simon Hugh, AdProTech Limited, Saffron Walden, Essex CB10 1XL (GB)
(74) Representative: Davies, Jonathan Mark
(86) International application number: PCT/GB2001/003034
(87) International publication number: WO 2002/004638

(56) References cited:
- WO-A-98/02454
- SIMONS K ET AL: "FUNCTIONAL RAFTS IN CELL MEMBRANES" NATURE, MACMILLAN JOURNALS LTD. LONDON, GB, vol. 387, 5 June 1997 (1997-06-05), pages 569-572, XP000876898 ISSN: 0028-0836 cited in the application
- PALMER ET AL: 'Staphylococcus aureus alpha-Toxin, Production of functionally intact site-specifically modifiable protein by introduction of cysteine at positions 69, 130, and 186' JOURNAL OF BIOLOGICAL CHEMISTRY vol. 268, no. 16, 05 June 1993, pages 11959 - 11962

## Description

This invention relates to fluorescent compounds capable of binding to lipid rafts on cells.

The plasma membrane of a cell forms a physical barrier that maintains the integrity of the cell and encapsulates the cytoplasm in which essential functions are carried out. The prevailing view of the structure of the cell membrane was described as the fluid mosaic model (Singer and Nicolson, *Science* **175,** 720, 1972) that suggested that the essential structural repeating unit is the phospholipid molecule in a bilayer arrangement with a thickness of about 500nm. In this model, membrane proteins are 'dissolved' in the bilayer and both lipids and proteins are distributed randomly in the membrane. However, it is now clear that different lipid species are distributed non-randomly over the exoplasmic and endoplasmic leaflets of the membrane (van Meer, *Annu. Rev. Cell Biol.* **5,** 247, 1989). In addition, lipids and proteins in the membrane are also organised in the lateral dimension into microdomains or so-called 'lipid rafts' (Simons and Ikonen, *Nature* **387,** 569, 1997).

The principal building blocks of cell membranes are phospholipids. These molecules are esters of glycerol comprising two fatty acyl residues (non-polar tails) and a single phosphate ester substituent (polar head group). Despite their overall similarity, natural phospholipids exhibit subtle differences in their fatty acid composition, degree of acyl chain unsaturation and in the type of polar head group (Dowhan, *Ann Rev Biochem.* **66,** 199, 1997). These differences can produce significant variations in the physical properties of membranes, the location of the phospholipids in the bilayer and in their biological activity. Phosphatidylcholine is distributed equally between the leaflets; by contrast, virtually all of the phosphatidylserine and most of the phosphatidylethanolamine and phosphatidylinositol resides on the cytoplasmic leaflet.

Sphingolipids are largely confined to the outer leaflet of the membrane. Sphingolipids differ from phospholipids because the structural backbone of these molecules is the lipophilic amino-dialcohol sphingosine, rather than glycerol. Sphingolipids include ceramides, sphingomyelins, and glycosphingolipids (glycosylceramides and gangliosides). Glycosphingolipids occur in the plasma membrane of all eukaryotic cells and interact at the cell surface with toxins, viruses and bacteria, as well as with receptors and enzymes and are involved in cell-type-specific cell adhesion processes. Gangliosides have complex oligosaccharide head groups containing at least one sialic acid residue in place of the single galactose or glucose residue of cerebrosides.

One further important constituent of biological membranes of mammals is cholesterol. Cholesterol is present on both leaflets of the membrane and intercalates among the fatty acyl chains, with its hydroxyl group oriented towards the aqueous surface and the aliphatic chain aligned parallel to the acyl chains in the centre of the bilayer. The presence of cholesterol in membranes has a significant effect of the physical properties of the membrane by restricting the freedom of movement of other membrane lipid components and decreasing the fluidity of the membrane. It is thought that the preferential packing of sphingolipids and cholesterol organizes the lipids into a liquid-ordered phase domain thus forming a so-called lipid raft (Rietveld and Simons, *Biochim. Biophys. Acta* **1376,** 467, 1998; Simons and Ikonen, *Nature* **387**, 569, 1997).
Sphingolipid-cholesterol rafts are insoluble in the detergent Triton X-100 at 4 degrees Celsius. Extraction under these conditions leads to the isolation of a membrane fraction termed a detergent-insoluble glycolipid-enriched complexes (DIGs). DIGs are thought to contain the remnants of the cellular raft domains that are aggregated together (Brown and Rose, *Cell* **68,** 533, 1992; Kurzchalia *et al., Trends Cell Biol.* **5,** 187, 1995; Parton and Simons, *Science* **269**, 1398, 1995). Several membrane proteins are specifically enriched in the DIG fraction and are considered to be raft proteins. The characterisation of proteins in DIGs has shown that proteins can selectively be included or excluded from these microdomains. Proteins bound to rafts include a class of proteins that are anchored to the exoplasmic leaflet of the membrane via a glycosylphosphatidylinositol (GPI) moiety (Brown and Rose, *Cell* **68,** 533, 1992) that contains two (usually) saturated fatty acyl chains. The association of GPI-anchored proteins with rafts is dependent on the length of acyl and alkyl chain composition (Benting *et al., FEBS Lett.* **462,** 47, 1999). Some cytoplasmic proteins are also found in the DIG fraction and are thus thought to be associated to raft domains via the cytoplasmic leaflet of the lipid bilayer. These include several signaling molecules such as G-alpha subunits of heterotrimeric G proteins or the doubly acylated Src-family kinases (Casey, *Science* **268**, 221, 1995). Investigation of the association of proteins with lipid rafts has shown that extraction of cholesterol by saponin abolishes the association with the DIG fraction as predicted from the involvement of cholesterol in the formation of lipid rafts (Cerneus *et al., J. Biol. Chem.* **268,** 3150, 1993; Hanada *et al., J. Biol. Chem.* **270,** 6254, 1995; Scheiffele *et al., EMBO J.* **16,** 5501, 1997).

The role of lipid rafts in cellular function is not fully understood. By studying the proteins and lipids that are found in DIGs several functions have been elucidated. Several signalling molecules partition into DIGs (Parton and Simons, *Science* **269**, 1398, 1995; Anderson, *Proc. Natl. Acad.* Sci (USA), **90,** 10909, 1993; Lisanti *et al., Trends Cell Biol.,* 4, 231, 1994) such as trimeric G proteins (Li *et al., J. Biol. Chem.,* **270,** 15693, 1995), src-family kinases (Casey, *Science* **268,** 221, 1995) and Ras (Song *et al., J. Biol. Chem.,* **271,** 9690, 1996; Mineo *et al., J. Biol. Chem.,* **271,** 11930, 1996). Lipids involved in signal transduction have also been localised in DIGs, including phosphoinositides (Hope and Pike, *Mol. Biol. Cell,* **7,** 843, 1996). Furthermore, the clustering of GPI-anchored proteins can activate different signalling pathways depending on the cell type. It is thought that the general function of lipid rafts in signal transduction is to concentrate receptors for interaction with ligands and effector proteins or lipids on both sides of the membrane and by modulating the association of proteins with rafts (Nykjaer *et al., J. Biol. Chem.* **269,** 25668, 1994; Field *et al., Proc. Natl. Acad. Sci.* (USA) **92**, 9201, 1995), to also help to ensure specificity and fidelity. In a further function, lipid rafts organise the transport of molecules from the endoplasmic reticulum to the cell surface (Simons and Ikonen, *Nature* **387,** 569, 1997).

Lipid rafts have also been characterised in living cells. The structure of lipid rafts has been studied by comparing the patching behaviour of different membrane proteins and lipids as detected by immunofluorescence microscopy (Harder *et al., J. Cell Biol.* **141,** 929, 1998). In this procedure, the proteins or lipids that are found in DIGs were crosslinked with fluorescently-labelled antibodies and/or cholera toxin. The membrane components formed patches on the cell surface. Patches formed by DIG-associated proteins, such as the GPI-anchored protein placental alkaline phosphatase, coincided with patches formed by the raft lipid ganglioside GM1 which was detected with the fluorescently-labelled B-subunit of cholera toxin. By contrast, the patches that were formed by proteins that were not DIG-associated were segregated from the patches of GPI-linked protein or GM1. Many other proteins have been characterised as raft-associated by this procedure (reviewed in Brown and London, *Annu. Rev. Cell Dev. Biol.* **14,** 111, 1998).
The organisation of proteins in cholesterol-dependent domains has also been analysed in living cells by fluorescence resonance energy transfer (FRET) microscopy between GPI-anchored proteins (Varma and Mayor, *Nature* **394,** 798, 1998) or by chemically crosslinking GPI-anchored proteins on the cell surface (Friedrichson and Kurzchalia, *Nature* **394**, 802, 1998). These studies showed that the GPI-anchored proteins cluster in membrane microdomains that are smaller than those seen after detergent extraction, and need cholesterol to be maintained. The exact size or protein and lipid composition of rafts in living cells is not fully understood.

WO98/02454 describes soluble derivatives of soluble polypeptides, which comprise two or more heterologous membrane binding elements with low membrane affinity covalently associated with the polypeptide, the elements being capable of interacting, independently and with thermodynamic additivity, with components of cellular or artificial membranes exposed to extracellular fluids.

We have now found that the combinatorial membrane binding element approach that was first described in WO98/02454 can be applied to localize compounds to lipid rafts. The present invention therefore provides compounds which, upon derivatisation with agents described in WO98/02454 and as herein, localise to lipid rafts and wherein the compound is a derivatised chemical or biological entity that possesses the physical property of fluorescence which enables lipid rafts to be identified and monitored. In these compounds, the membrane-binding elements described in WO98/02454 interact selectively with components of lipid rafts including but not restricted to phosphatidylserine, phosphatidyl glycerol, glycosphingolipids, cholesterol, GPI-anchored proteins associated with lipid rafts and other protein components of lipid rafts that may be found normally on the exoplasmic face of the cell.

WO98/02454 provides a variety of methods for attachment of the soluble polypeptide to the membrane binding elements. As an example, the linkage of these two components was provided by a disulphide bond formed by the reaction of a pyridylthio group on one component with a thiol group on the other component. The thiol group may be a native thiol or one introduced as a protein attachment group (described therein). Alternatively, the protein attachment group can contain a thiol-reactive entity such as the 6-maleimidohexyl group.

Soluble forms of proteins that are normally located in lipid rafts can be produced by either recombinant means or in certain cases purified from a biological source such as human urine or plasma. Such materials may include, but are not restricted to GPI-anchored proteins. These proteins may be treated with a reagent such as 2-iminothiolane (described in WO98/0245 and herein). This procedure introduces one or more protein attachment groups into the protein. The modified protein may be separated from excess modifying agents by standard techniques such as dialysis, ultrafiltration, gel filtration and solvent or salt precipitation. The intermediate material may be stored in frozen solution or lyophilised. The modified protein may be then reacted further with a pyridylthio group that is linked to a membrane binding peptide. In the above process, there can be no guarantee that the chemical modification of the protein by 2-iminothiolane or another thiolating or thiol-reactive agent would produce a polypeptide that retained biological activity. Furthermore, there can be no guarantee that the soluble form of the lipid raft protein which has been chemically linked to entities described in WO98/02454 (which are structurally quite different from GPI anchors) would retain the same biological activity as the protein that has been extracted from a lipid raft.

Similar procedures to those described above may also be applied to soluble proteins that do not normally localize in lipid rafts.

In an aspect of the invention, the membrane binding peptide may be linked to a terminal cysteine residue which has been introduced into a protein by recombinant methods. The polypeptide portion of the derivatives of the invention may be prepared by expression in suitable hosts of modified genes encoding the soluble polypeptide of interest plus one or more peptidic membrane binding elements and optional residues such as cysteine to introduce linking groups to facilitate post translational derivatisation with additional membrane binding elements.

A process for preparing a derivative according to the invention which process comprises expressing DNA encoding the polypeptide portion of said derivative in a recombinant host cell and recovering the product and thereafter post translationally modifying the polypeptide to chemically introduce membrane binding elements with selectivity for lipid rafts..

In particular, the recombinant aspect of the process may comprise the steps of:
i) preparing a replicable expression vector capable, in a host cell, of expressing a DNA polymer comprising a nucleotide sequence that encodes said polypeptide portion;
ii) transforming a host cell with said vector;
iii) culturing said transformed host cell under conditions permitting expression of said DNA polymer to produce said polypeptide; and
iv) recovering said polypeptide.

The recombinant process of the invention may be performed by conventional recombinant techniques such as described in Sambrook *et al.,* Molecular Cloning : A laboratory manual 2nd Edition. Cold Spring Harbor Laboratory Press (1989) and DNA Cloning vols I, II and III (D. M. Glover ed., IRL Press Ltd).

The preparation may be carried out chemically, enzymatically, or by a combination of the two methods, *in vitro* or *in vivo* as appropriate. Thus, the DNA polymer may be prepared by the enzymatic ligation of appropriate DNA fragments, by conventional methods such as those described by D. M. Roberts *et al., Biochemistry* **24,** 5090, 1985.

The DNA fragments may be obtained by digestion of DNA containing the required sequences of nucleotides with appropriate res triction enzymes, by chemical synthesis, by enzymatic polymerisation, or by a combination of these methods.

Digestion with restriction enzymes may be performed in an appropriate buffer at a temperature of 20°-70°C, generally in a volume of 50ml or less with 0.1-10mg DNA. Enzymatic polymerisation of DNA may be carried out *in vitro* using a DNA polymerase such as DNA polymerase 1 (Klenow fragment) in an appropriate buffer containing the nucleoside triphosphates dATP, dCTP, dGTP and dTTP as required at a temperature of 10°-37°C, generally in a volume of 50ml or less.

Enzymatic ligation of DNA fragments may be carried out using a DNA ligase such as T4 DNA ligase in an appropriate buffer at a temperature of 4°C to 37°C, generally in a volume of 50ml or less.

The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J.Gait, H.W.D. Matthes M. Singh, B.S. Sproat and

R.C. Titmas, Nucleic Acids Research, 1982, 10, 6243; B.S. Sproat and W. Bannwarth, Tetrahedron Letters, 1983,24, 5771; M.D. Matteucci and M.H. Caruthers, Tetrahedron Letters, 1980, 21, 719; M.D. Matteucci and M.H. Caruthers, Journal of the American Chemical Society, 1981,103, 3185; S.P. Adams *et al.,* Journal of the American Chemical Society, 1983, 105, 661; N.D. Sinha, J. Biernat, J. McMannus and H. Koester, Nucleic Acids Research, 1984, 12, 4539; and H.W.D. Matthes *et al.,* EMBO Journal, 1984, 3, 801. Preferably an automated DNA synthesiser (for example, Applied Biosystems 381A Synthesiser) is employed.

The DNA polymer is preferably prepared by ligating two or more DNA molecules which together comprise a DNA sequence encoding the polypeptide.

The DNA molecules may be obtained by the digestion with suitable restriction enzymes of vectors carrying the required coding sequences.

The precise structure of the DNA molecules and the way in which they are obtained depends upon the structure of the desired product. The design of a suitable strategy for the construction of the DNA molecule coding for the polypeptide is a routine matter for the skilled worker in the art.

In particular, consideration may be given to the codon usage of the particular host cell. The codons may be optimised for high level expression in *E. coli* using the principles set out in *Devereux et al.,* (1984) Nucl. Acid Res., **12,** 387.

The expression of the DNA polymer encoding the polypeptide in a recombinant host cell may be carried out by means of a replicable expression vector capable, in the host cell, of expressing the DNA polymer. Novel expression vectors also form part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the polypeptide, under ligating conditions.

The ligation of the linear segment and more than one DNA molecule may be carried out simultaneously or sequentially as desired.

Thus, the DNA polymer may be preformed or formed during the construction of the vector, as desired. The choice of vector will be determined in part by the host cell, which may be prokaryotic, such as *E*. *coli,* or eukaryotic, such as mouse C127, mouse myeloma, chinese hamster ovary, fungi e.g. filamentous fungi or unicellular 'yeast' or an insect cell such as Drosophila. The host cell may also be in a transgenic animal. Suitable vectors include plasmids, bacteriophages, cosmids and recombinant viruses derived from, for example, baculoviruses or vaccinia.

The DNA polymer may be assembled into vectors designed for isolation of stable transformed mammalian cell lines expressing the fragment e.g. bovine papillomavirus vectors in mouse C127 cells, or amplified vectors in chinese hamster ovary cells (DNA Cloning Vol. II D.M. Glover ed. IRL Press 1985; Kaufman, R.J. *et al..* Molecular and Cellular Biology 5, 1750-1759, 1985; Pavlakis G.N. and Hamer, D.H. Proceedings of the National Academy of Sciences (USA) 80, 397-401, 1983; Goeddel, D.V. *et al.,*European Patent Application No. 0093619, 1983).

The preparation of the replicable expression vector may be carried out conventionally with appropriate enzymes for restriction, polymerisation and ligation of the DNA, by procedures described in, for example, Sambrook *et al.,* cited above. Polymerisation and ligation may be performed as described above for the preparation of the DNA polymer. Digestion with restriction enzymes may be performed in an appropriate buffer at a temperature of 20°-70°C, generally in a volume of 50ml or less with 0.1-10mg DNA.

The recombinant host cell is prepared, in accordance with the invention, by transforming a host cell with a replicable expression vector of the invention under transforming conditions. Suitable transforming conditions are conventional and are described in, for example,

*Sambrook et al.,* cited above, or "DNA Cloning" Vol. II, D.M. Glover ed., IRL Press Ltd, 1985.

The choice of transforming conditions is determined by the host cell. Thus, a bacterial host such as *E. coli,* may be treated with a solution of CaCl2 (Cohen *et al.*, Proc. Nat. Acad. Sci., 1973, 69, 2110) or with a solution comprising a mixture of RbCl, MnC12, potassium acetate and glycerol, and then with 3-[N-morpholino]-propane-sulphonic acid, RbCl and glycerol or by electroporation as for example described by Bio-Rad Laboratories, Richmond, California, USA, manufacturers of an electroporator. Mammalian cells in culture may be transformed by calcium co-precipitation of the vector DNA onto the cells or by using cationic liposomes.

The invention also extends to a host cell transformed with a replicable expression vector of the invention.

Culturing the transformed host cell under conditions permitting expression of the DNA polymer is carried out conventionally, as described in, for example, Sambrook *et al.,* and "DNA Cloning" cited above. Thus, preferably the cell is supplied with nutrient and cultured at a temperature below 45°C.

The protein product is recovered by conventional methods according to the host cell. Thus, where the host cell is bacterial such as *E*. *coli* and the protein is expressed intracellularly, it may be lysed physically, chemically or enzymatically and the protein product isolated from the resulting lysate. Where the host cell is mammalian, the product is usually isolated from the nutrient medium.

Where the host cell is bacterial, such as *E. coli,* the product obtained from the culture may require folding for optimum functional activity. This is most likely if the protein is expressed as inclusion bodies. There are a number of aspects of the isolation and folding process that are regarded as important. In particular, the polypeptide is preferably partially purified before folding, in order to minimise formation of aggregates with contaminating proteins and minimise misfolding of the polypeptide. Thus, the removal of contaminating *E. coli* proteins by specifically isolating the inclusion bodies and the subsequent additional purification prior to folding are important aspects of the procedure.

The folding process is carried out in such a way as to minimise aggregation of intermediate-folded states of the polypeptide. Thus, careful consideration needs to be given to, among others, the salt type and concentration, temperature, protein concentration, redox buffer concentrations and duration of folding. The exact condition for any given polypeptide generally cannot be predicted and must be determined by experiment.

There are numerous methods available for the folding of proteins from inclusion bodies and these are known to the skilled worker in this field. The methods generally involve breaking all the disulphide bonds in the inclusion body, for example with 50mM 2-mercaptoethanol, in the presence of a high concentration of denaturant such as 8M urea or 6M guanidine hydrochloride. The next step is to remove these agents to allow folding of the proteins to occur. Formation of the disulphide bridges requires an oxidising environment and this may be provided in a number of ways, for example by air, or by incorporating a suitable redox system, for example a mixture of reduced and oxidised glutathione.

Preferably, the inclusion body is solubilised using 8M urea, in the presence of mercaptoethanol, and protein is folded, after initial removal of contaminating proteins, by addition of cold buffer. Suitable buffers may be identified using the techniques described in LDodd *et al,* 'Perspectives in Protein Engineering and Complementary Technologies' , Mayflower Publications, 66-69, 1995. A suitable buffer for many of the SCR constructs described herein is 20mM ethanolamine containing 1mM reduced glutathione and 0.5mM oxidised glutathione. The folding is preferably carried out at a temperature in the range 1 to 5oC over a period of 1 to 4 days.

If any precipitation or aggregation is observed, the aggregated protein can be removed in a number of ways, for example by centrifugation or by treatment with precipitants such as ammonium sulphate. Where either of these procedures are adopted, monomeric polypeptide is the major soluble product.

If the bacterial cell secretes the protein, folding is not usually necessary.

The polypeptide portion of the derivative of the invention may include a C-terminal cysteine to facilitate post translational modification. A soluble polypeptide including a C-terminal cysteine also forms part of the invention. Expression in a bacterial system is preferred for proteins of moderate size (up to ~70kDa) and with <~8 disulphide bridges. More complex proteins for which a free terminal Cys could cause refolding or stability problems may require stable expression in mammalian cell lines (especially CHO). This will also be needed if a carbohydrate membrane binding element is to be introduced post-translationally. The use of insect cells infected with recombinant baculovirus encoding the polypeptide portion is also a useful general method for preparing more complex proteins and will be preferred when it is desired to carry out certain post-translational processes (such as palmitoylation) biosynthetically (see for example, M.J.Page *et al* J.Biol.Chem. 264, 19147-19154, 1989)

A preferred method of handling proteins C-terminally derivatised with cysteine is as a mixed disulphide with mercaptoethanol or glutathione or as the 2-nitro, 5-carboxyphenyl thio-derivative as generally described below in Methods.

Peptide membrane binding elements may be prepared using standard solid state synthesis such as the Merrifield method and this method can be adapted to incorporate required non-peptide membrane binding elements such as N-acyl groups derived from myristic or palmitic acids at the N terminus of the peptide. In addition activation of an amino acid residue for subsequent linkage to a protein can be achieved during chemical synthesis of such membrane binding elements. Examples of such activations include formation of the mixed 2-pyridyl disulphide with a cysteine thiol or incorporation of an N-haloacetyl group. Peptides can optionally be prepared as the C-terminal amide.

After the linkage reaction, the polypeptide conjugate can be isolated by a number of chromatographic procedures described in WO98/02454. The conjugate may be characterized by a number of techniques including high performance gel filtration, SDS polyacrylamide gel electrophoresis, isoelectric focussing, or mass spectrometry.

The following Methods and Examples illustrate the invention.

### GENERAL METHODS USED IN EXAMPLES

### (i) DNA Cleavage

Cleavage of DNA by restriction endonucleases was carried out according to the manufacturer's instructions using supplied buffers. Double digests were carried out simultaneously if the buffer conditions were suitable for both enzymes. Otherwise double digests were carried out sequentially where the enzyme requiring the lowest salt condition was added first to the digest. Once the digest was complete the salt concentration was altered and the second enzyme added.

### (ii) DNA ligation

Ligations were carried out using T4 DNA ligase purchased from Promega, as described in Sambrook *et al,* (1989) Molecular Cloning: A Laboratory Manual 2nd Edition. Cold Spring Harbour Laboratory Press.

### (iii) Plasmid isolation

Plasmid isolation was carried out by the alkaline lysis method described in Sambrook *et al,* (1989) Molecular Cloning: A Laboratory Manual 2nd Edition. Cold Spring Harbour Laboratory Press or by one of two commercially available kits: the Promega WizardTM *Plus* Minipreps or Qiagen Plasmid Maxi kit according to the manufacturer's instructions.

### (iv) DNA fragment isolation

DNA fragments were excised from agarose gels and DNA extracted using one of three commercially available kits: the QIAEX ge1 extraction kit or Qiaquick gel extraction kit (QIAGEN Inc., USA), or GeneClean (Bio 101 Inc, USA) according to the manufacturer's instructions.

### (v) Introduction of DNA into E. coli

Plasmids were transformed into *E. coli* XL1-Blue (Stratagene), HMS174(DE3) (Novagen, UK) or UT5600(DE3) (see below) that had been made competent using calcium chloride as described in Sambrook *et al,* (op.cit.). UT5600 was purchased from New England Biolabs (#801-I) and was converted to a DE3 lysogen by Dr A. Topping, Zeneca Life Science Molecules, Billingham UK. UT5600 was isolated as a mutant of K12 strain RW193 (itself derived from AB1515) which was insensitive to colicin -B (McIntosh et al. (1979) J.Bact. 137 p653). It was not initially known that *ompT* had been lost, but further work by the same group showed that protein a (now *OmpT*) was lacking (Earhart et al (1979) FEMS Micro Letts 6 p277). The nature of the mutation was determined to be a large deletion (Elish et al (1988) J Gen Micro **134** 1355.

### (vi) DNA sequencing

DNA sequencing was contracted out to Lark (Saffron Walden, Essex UK) or MWG (Milton Keynes, UK).

### (vii) Production of oligonucleotides

Oligonucleotides were purchased from Cruachem (UK) or Genosys-Sigma (Pampisford, Cambridgeshire UK)

### (viii) Polymerase chain reaction amplification of DNA

Purified DNA or DNA fragments from ligation reactions or DNA fragments excised and purified from agarose gels were amplified by PCR from two primers complementary to the 5' ends of the DNA fragment: Approximately 0.1 - 1 microg of DNA was mixed with commercially available buffers for PCR amplification such as 10 mM Tris pH 8.3 (at 25oC), 50 mM KCl, 0.1% gelatin; MgC12 concentrations were varied from 1.5 mM to 6 mM to find a suitable concentration for each reaction. Oligonucleotide primers were added to a final concentration of 2 microM; each dNTP was added to a final concentration of 0.2 mM. 1 unit of Taq DNA polymerase was then added to the reaction mixture (purchased from a commercial source, e.g. Gibco). The final reaction volume varied from 20 microL to 100 microL, which was overlayed with mineral oil to prevent evaporation. Thermal cycling was then started on a thermal cycler such as the PCR machine from MJ Research. A typical example of conditions used was 94oC for 5 minute, 55oC for 1 minute, and 72 oC for 2 minutes; however, the optimal temperatures for cycling can be determined empirically by workers skilled in the art. The DNA fragment was amplified by repeating this temperature cycle for a number of times, typically 30 times.

### (ix) Colorimetric determination of protein concentration

Protein concentration determination utilised a colorometric method utilising Coomassie Plus Protein Assay Reagent (Pierce Chemical Company) according to the manufacturer's instructions. The assay used an APT154 reference standard prepared using similar methodology to that described in WO 98/02454, Example 6.

### (x) Sodium dodecyl sulphate polyacrylamide gel electrophoresis (SDS PAGE)

SDS PAGE was carried out generally using the Novex system (Invitrogen) according to the manufacturer's instructions. Prepacked gels of 4 - 20% acrylamide were usually used.

Samples for electrophoresis, including protein molecular weight standards (for example LMW Kit, Pharmacia or Novex Mark 12) were usually diluted in 1%(w/v) SDS - containing buffer (with or without 5%(v/v) 2-mercaptoethanol), and left at room temperature for about 10 to 30 min before application to the gel.

### (xi) Reduction of disulphides and modification of thiols in proteins

There are a number of methods used for achieving the title goals. The reasons it may be necessary to carry out selective reduction of disulphides is that during the isolation and purification of multi-thiol proteins, in particular during refolding of fully denatured multi-thiol proteins, inappropriate disulphide pairing can occur. In addition, even if correct disulphide paring does occur, it is possible that a free cysteine in the protein may become blocked, for example with glutathione or cysteine. These derivatives are generally quite stable. In order to make them more reactive, for example for subsequent conjugation to another functional group, they need to be selectively reduced, with for example dithiothreitol (DTT) or Tris (2-carboxyethyl) phosphine.HCl (TCEP) then optionally modified with a function which is moderately unstable. An example of the latter is Ellmans reagent (DTNB) which gives a mixed disulphide. In the case where treatment with DTNB is omitted, careful attention to experimental design is necessary to ensure that dimerisation of the free thiol-containing protein is minimised. Reference to the term 'selectively reduced' above means that reaction conditions eg. duration, temperature, molar ratios of reactants have to be carefully controlled so that reduction of disulphide bridges within the natural architecture of the protein is minimised. All the reagents are commercially available eg. from Sigma or Pierce.

The following general examples illustrate the type of conditions that may be used and that are useful for the generation of free thiols and their optional modification. The specific reaction conditions to achieve optimal thiol reduction and/or modification are ideally determined for each protein batch.

TCEP may be prepared as a 20mM solution in 50mM Hepes (approx. pH 4.5) and may be stored at -40 degrees C. DTT may be prepared at 10mM in sodium phosphate pH 7.0 and may be stored at -40 degrees C. DTNB may be prepared at 10mM in sodium phosphate pH 7.0 and may be stored at -40 degrees C. All of the above reagents are typically used at molar equivalence or molar excess, the precise concentrations ideally identified experimentally. The duration and the temperature of the reaction are similarly determined experimentally. Generally the duration would be in the range 1 to 24 hours and the temperature would be in the range 2 to 30 degrees C. Excess reagent may be conveniently removed by buffer exchange, for example using Sephadex G25. A suitable buffer is 0.1M sodium phosphate pH7.0.

### Fluorescent Labeling of Proteins

Two antibodies were labelled with fluorophores to provide two fluorescent probes that react with cellular components that localise at the cell surface in lipid rafts. The rat monoclonal anti-CD59 antibody YTH 53.1 (0.75mg; Davies *et al., J. Exp. Med.* **170,** 637, 1989) in PBS was labelled using the Alexa 546 Protein Labeling Kit (Molecular Probes Inc, Oregon, USA) according to the manufacturer's instructions. The final molar ratio of Alexa 546 to antibody was 3.4:1. A rabbit polyclonal antibody that binds to the B subunit of cholera toxin (1mg; Biogenesis, Dorset, UK) was labelled using the FluoroLink-Ab Cy2 Labeling Kit (Amersham Pharmacia Biotech, Little Chalfont, UK) according to the manufacturer's instructions. The final molar ratio of Cy2 to antibody was 2.2:1.

To detect APT070 on the cell surface 1mg of a mouse monoclonal antibody, 3e10, raised against the first three short consensus repeat domains of human CR1 (CD35) was fluorescently labelled using the FluoroLink-Ab Cy3 Labeling Kit (Amersham Pharmacia Biote ch, Little Chalfont, UK) according to the manufacturer's instructions. The final molar ratio of Cy3 to antibody was approximately 6:1.

### Fluorescence Microscopy

Fluorescence microscopy was used to characterise the distribution of various cellular components that localise at the cell surface in lipid rafts. The components that were chosen were the ganglioside GM_{1,} a major constituent of lipid rafts, and the GPI-anchored protein CD59. GM₁ was detected using the B subunit of cholera toxin conjugated to FTTC (FITC-CTB; Sigma-Aldrich Chemical Co., Gillingham, UK) and a fluorescently labelled cholera toxin B-subunit antibody described in (xvii). Endogenous CD59 was detected with the fluorescently labelled antibody YTH53.1 described in (xvii) above. 15 ml of Raji cells were grown in suspension culture were harvested and washed twice in PBS and finally resuspended in 1ml of PBS. The cells were counted on a haemocytometer. For all incubations non-stick microtubes were used (Anachem, Luton, UK). A total of 501 of cells was used and contained 5x10⁵ Raji cells. These cells were then incubated with a variety of compounds (See Example 13 for specific incubation details). After fixation, 10 microlitres of cells were mounted on a glass slide and a coverslip was placed on top of the sample, air excluded, and finally the edges of the coverslip were sealed with clear nail varnish. Samples were viewed using an epifluorescence microscope (Nikon E400, Surrey, UK). Fluorescence was visualised using the following filters; for green fluorescence a FITC filter, excitation wavelength 465-495nm; and for red fluorescence, a G2A filter, excitation wavelength 510-560nm.

Non-specific interactions between the Cy-3 labeled 3e10 antibody and the Cy-2 labeled anti-CTB were not detected. Furthermore, for each fluorescent marker used, there was no crossover between the red and green fluorescence using the FITC and G2A filter.

### Confocal Microscopy

For confocal microscopy, cells were treated with a variety of compounds and antibodies as described above. The cells were fixed and mounted as described and visualised using an Axiophot microscope (Carl Zeiss) coupled to a Colour Coolview CCD colour camera. Filter settings for the simultaneous detection of red and green fluorescence were used. The digital images were processed using Photoshop software (Adobe Systems). Cell images were obtained that visualised either the surface of the cell or cell sections. These latter images revealed the localisation of compounds intracellularly.
Example 1. A method for the synthesis of the free cysteine form of a membrane-localising agent (APT544)
Example 2. A method for the synthesis of the lipid-raft targeted fluorescent probe APT2087
Example 3. A method for the synthesis of the lipid-raft targeted fluorescent probe APT2104
Example 4. A method for the synthesis of the lipid-raft targeted fluorescent probe APT2105
Example 5. Demonstration by fluorescence microscopy of colocalisation of proteins modified with membrane-targeting peptides and known lipid raft markers
Example 6. Demonstration by confocal microscopy of the intracellular localization of lipid raft-targeted compounds
Example 7. Demonstration of lysosomal localisation of APT2104 in cultured cells.

### Example 1 A method for the synthesis of the free cysteine form of a membrane-localising agent: preparation of N-myristoyl-Gly-Ser-Ser-Lys-Ser-Pro-Ser-Lys-Lys-Lys-Lys-Lys-Lys-Pro-Gly-Glu-Cys-NH₂ (APT544; Seq. ID No. 12)

APT542 (250 µL of a 21.6 mM solution in DMSO) And TCEP (400 µL of a 100 mM solution in water) were mixed. Dethiopyridylation was monitored by HPLC (10-90% acetonitrile in 0.1% TFA) and evidenced by the disappearance of APT542 at 13.9 mins and the appearance of APT544 at 14.2 mins. After 2 h the reaction mixture was purified by preparative HPLC. The product-containing fractions were taken to low volume on the rotary evaporator and APT544 obtained as a white solid after lyophilisation. Treatment of an aqueous solution of APT544 with 1 mM DTT yielded no increase in absorbance at 343 nm indicating complete removal of the thiopyridyl function.

### Example 2 A method for the synthesis of a lipid-raft targeted fluorescent probe (1): preparation of N-Myristoyl-Gly-Ser-Ser-Lys-Ser-Pro-Ser-Lys-Lys-Lys-Lys-Lys-Lys-Pro-Gly-Glu-Cys(S-5-succinimido fluorescein)-NH₂ (APT2087; Seq. ID No. 13)

APT544 (3.0 mg, 1.50 µmol) was dissolved in degassed 20 mM sodium phosphate, 150 mM NaCl, pH 7.2, 1 mM EDTA (500 µL) and fluorescein-5-maleimide (4.65 µmol, 2 mg in 100 µL DMF) added in one portion. The mixture was stirred at 4°C in the dark overnight. Reaction completion was monitored by HPLC, evidenced by disappearance of APT 544 at 12.8 mins and the appearance APT 2087 at 14.5 mins. The reaction was purified and lyophilised as before to yield a yellow solid.

### Example 3. A method for the synthesis of the lipid-raft targeted fluorescent probe (2): Synthesis of N-Myristoyl-Gly-Ser-Ser-Lys-Ser-Pro-Ser-Lys-Lys-Lys-Lys-Lys-Lys-Pro-Gly-Glu-Cys(S-succinimido Alexafluor™488 C₅)-NH₂ (APT2104; Seq. ID No. 14)

APT544 (2.40 mg, 1.20 µmol) was dissolved in degassed 20 mM sodium phosphate, 150 mM NaCl, pH 7.2, 1 mM EDTA (500 µL). Alexafluor™488 C₅ maleimide (1 mg, 1.39 µmol, Molecular Probes Inc, Oregon, USA) was dissolved in DMSO (30 µL) and added in one portion to the APT544 solution. The mixture was stirred at 4°C in the dark overnight, purified and lyophilised as before to yield the title compound as an orange solid. The retention time was identical to that of APT544 (14.2 mins) but unlike APT 544, APT2104 absorbed strongly at 343 nm.

### Example 4. A method for the synthesis of the lipid-raft targeted fluorescent probe (3): preparation of N-Myristoyl-Gly-Ser-Ser-Lys-Ser-Pro-Ser-Lys-Lys-Lys-Lys-Lys-Lys-Pro-Gly-Glu-Cys(S-succinimido Alexafluofluor™546 C₅)-NH₂ (APT2105; Seq. ID No. 15)

APT544 (2.40 mg, 1.20 µmol) Was dissolved in degassed 20 mM sodium phosphate, 150 mM NaCl, pH 7.2, 1 mM EDTA (500 µL). Alexafluor™546 C₅ maleimide (1 mg, 1.03 µmol, source as above) was dissolved in DMSO (30 µL) and added in one portion to the APT544 solution. The mixture was stirred at 4°C in the dark overnight, purified and lyophilised as before to yield the title compound as a purple solid. RT 15.7 mins; MALDI Mass Spec: C₁₃₁H₂₀₅Cl₃N₂₉O₃₆S₄ requires 2997.83 Da; Observed 3000 Da.

### Example 5. Demonstration by fluorescence microscopy of colocalisation of-proteins modified with membrane-targeting peptides and known lipid raft markers

Commonly used markers of lipid rafts are cholera toxin B subunit (CTB) that binds specifically to the ganglioside GM₁, itself a major constituent of lipid rafts; and CD59, which was used in this study as an example of a GPI anchored protein. These raft-associated markers were found to co-localise with three different compounds: the derivatized fluorophores APT2104 and APT2105; and APT070, a derivative of a protein that is not normally associated with lipid rafts.

### (i) Endogenous CD59 and APT2104:

Endogenous CD59 was detected with the monoclonal antibody YTH53.1 that had been labeled with Alexa Fluor 546 as described in (xvii, herein). 2 microlitre of labeled-YTH53.1 antibody (7 micromolar in PBS) and 2 microlitre of APT2104 (22micromolar in PBS) were added to Raji cells as described in (xviii) and incubated for 60min and 37C. The cells were washed three times with 0.5ml of ice-cold PBS. The cells were then fixed in 50 microlitre of 4%(wlv) paraformaldehyde, 0.125%(v/v) glutaraldehyde for 10min at 22C. The cells were washed with 0.5ml ice-cold PBS and finally resuspended in 50 microlitre of PBS. The cells were examined by fluorescence microscopy as described in section xviii.

### (ii) Cholera Toxin B subunit and APT2105

2 microlitre of FITC-CTB (100 micromolar in PBS), and 2 microlitre of APT2015 (20 micromolar in PBS) were added to the Raji cells as described in (xviii) and incubated for 60min at 37C. The cells were washed three times with 0.5ml of ice-cold PBS. To enhance the weak fluorescence signal from of the cholera toxin, the cells were resuspended in 48 microlitre of PBS and 2 microlitre of Cy2-anti cholera toxin B subunit antibody (6 micromolar; described in xvii above) and incubated for 60min at 37C. The cells were washed three times with 0.5ml of ice-cold PBS. The cells were then fixed in 50 microlitre of 4%(w/v) paraformaldehyde, 0.125%(v/v) glutaraldehyde for 10min at 22C. The cells were washed with 0.5ml ice-cold PBS and finally resuspended in 50 microlitre of PBS. The cells were examined by fluorescence microscopy as described in section xviii.

### (iii) APT070 and Cholera Toxin B subunit

2 microlitre of FITC-CTB (100 micromolar in PBS) and 0.5microlitre of APT070 (100 micromolar in PBS; described in WO 98/02454; [SCR1-3]-Cys-S-S-[MSWP-1]; example 8) were added to the cell suspension and incubated for 60min at 37C. The cells were washed three times with 0.5ml of ice-cold PBS. To enhance the weak fluorescence signal from of the cholera toxin and to detect the APT070 on the cell surface, the cells were resuspended in 46 microlitre of PBS and 2 microlitre of both Cy2-anti cholera toxin B subunit antibody (6 micromolar) and Cy3-labelled 3e10 was added and incubated for 60min at 37C. The cells were washed three times with 0.5ml of ice-cold PBS. The cells were fixed in 50 microlitre of 4%(w/v) paraformaldehyde, 0.125%(v/v) glutaraldehyde for 10min at 22C. The cells were washed with 0.5ml ice-cold PBS and finally resuspended in 50 microlitre of PBS. The cells were examined by fluorescence microscopy as described in section xviii.

In the above experiments a discrete punctate pattern was seen in each; of APT2105 with FITC-CTB and of endogenous CD59 with APT2104 and also APT070 with FITC-CTB on Raji cells. These patterns showed a very similar distribution of fluorescence between the targeted proteins and the markers for lipid rafts. This provides strong evidence that modification with the membrane targeting peptide APT542 confers selective binding to lipid rafts within the cell membrane.

### Example 6. Demonstration by confocal microscopy of the intracellular localization of lipid raft-targeted compounds

This example follows the same procedure as described in example 13 with the following modifications. In certain experiments, lipid raft targeted compounds were added to the cells either singly, or in combination with other compounds. The compounds were then visualized with fluorescent antibodies or in the case of APT2104 and APT2105 by their intrinsic fluorescence, in each case using a confocal microscope as described in methods. In the following cases intracellular fluorescence was seen deriving from the lipid raft targeting peptides: Endogenous CD59 and APT2104; FITC-CTB and APT2105; APT070 and FITC-CTB; APT2104; APT2105. These data demonstrate that the derivatisation of compounds with peptides that localize the compound to a lipid raft also deliver the compound intracellularly.

### Example 7: Demonstration of lysosomal localisation of APT2104 in cultured cells.

COS-7 cells were grown to confluence on poly-D-lysine -coated coverslips. Cells were washed once with HEPES-buffered DMEM, and were subsequently kept in this medium. APT2104 was added at concentrations in the range 0.1-1.0 µM and the cells were incubated for different times at 37 °C. For lysosomal staining, 75 nM 'Lysotracker Red' (Molecular Probes Inc.) was added to the cells for the final hour of the incubation. Cells were then washed three times in DMEM and either viewed live in a cavity slide by fluorescence microscopy or else fixed with paraformaldehyde (as described above) prior to mounting and imaging by fluorescence microscopy.

### Chemical Structures

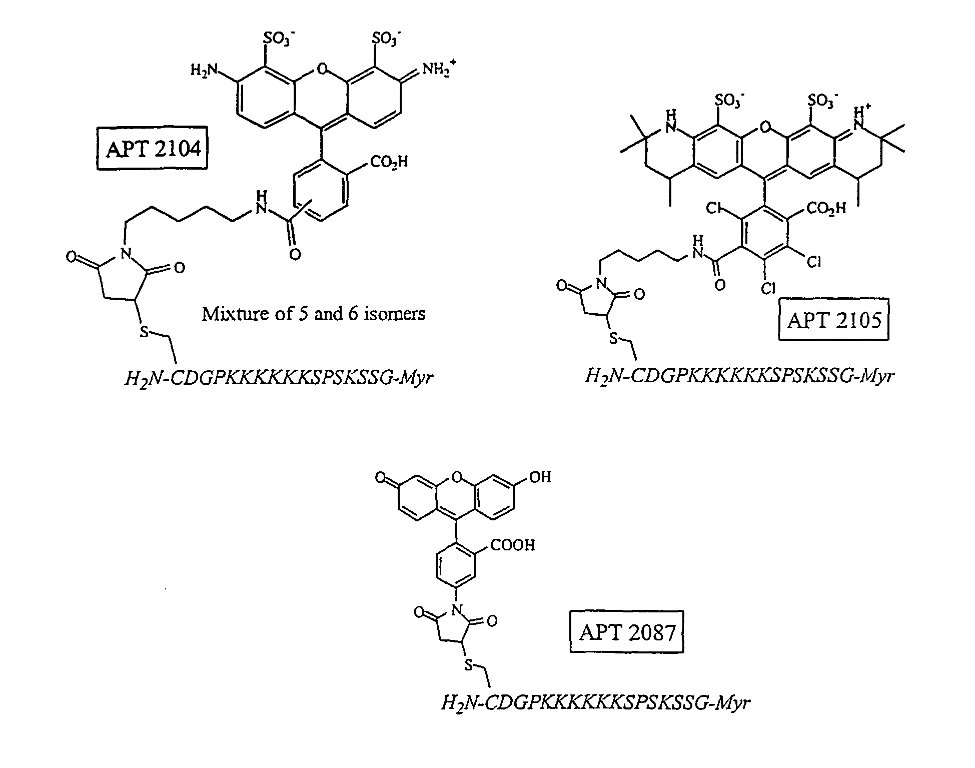

### SEQUENCE LISTING

(2) INFORMATION FOR SEQ ID NO:1:
   (i) SEQUENCE CHARACTERISTICS:
      (A) LENGTH: 77 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO: 1:
(2) INFORMATION FOR SEQ ID NO:2:
   (i) SEQUENCE CHARACTERISTICS:
      (A) LENGTH: 17 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:2:
      N-(myristoyl) - Gly-Ser-Ser-Lys-Ser-Pro-Ser-Lys-Lys-Lys-Lys-Lys-Lys-Pro-Gly-Asp-Cys-(2-thiopyridyl)-NH2
(2) INFORMATION FOR SEQ ID NO:3:
   (i) SEQUENCE CHARACTERISTICS:
      (A) LENGTH: 70 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 82 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 83 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 71 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 99 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 100 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 88 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 254 amino acids
      (B) TYPE: amino acid
      (D) TOPOLOGY: linear
   (ii) MOLECULE TYPE: peptide
   (v) FRAGMENT TYPE:
   (xi) SEQUENCE DESCRIPTION: SEQ ID NO:10
(2) INFORMATION FOR SEQ ID NO: 11:
   (i) SEQUENCE CHARACTERISTICS:
      (A) LENGTH: 271 amino acids
      (B) TYPE: amino acid
      (D) TOPOLOGY: linear, 2 polypeptide chains, disulphide linked
   (ii) MOLECULE TYPE: peptide
   (v) FRAGMENT TYPE:
   (xi) SEQUENCE DESCRIPTION: SEQ ID NO: 11

### SEQUENCE LISTING

<110> AdProTech Limited
<120> Compounds Targeted to Cellular Locations
<130> JMD/42008/PCT
<160> 15
<170> PatentIn version 3.1
<210> 1
   <211> 77
   <212> PRT
   <213> Homo sapiens
<400> 1
   Leu Gln Cys Tyr Asn Cys Pro Asn Pro Thr Ala Asp Cys Lys Thr Ala
   1               5                   10                  15
   Val Asn Cys Ser Ser Asp Phe Asp Ala Cys Leu Ile Thr Lys Ala Gly
               20                  25                  30
   Leu Gln Val Tyr Asn Lys Cys Trp Lys Phe Glu His Cys Asn Phe Asn
           35                  40                  45
   Asp Val Thr Thr Arg Leu Arg Glu Asn Glu Leu Thr Tyr Tyr Cys Cys
       50                  55                  60
   Lys Lys Asp Leu Cys Asn Phe Asn Glu Gln Leu Glu Asn
   65                  70                  75
<210> 2
   <211> 17
   <212> PRT
   <213> Artificial Sequence
<220>
   <223> Synthetic polypeptide
<220>
   <221> SITE
   <222> (1)..(1)
   <223> N-(myristoyl) attached to Gly1
<220>
   <221> SITE
   <222> (17)..(17)
   <223> (2-thiopyridyl)-NH2 attached to Cys17
<400> 2
   Gly Ser Ser Lys Ser Pro Ser Lys Lys Lys Lys Lys Lys Pro Gly Asp
   1               5                   10                  15
   Cys
<210> 3
   <211> 70
   <212> PRT
   <213> Artificial Sequence
<220>
   <223> Synthetic polypeptide
<400> 3
   Leu Gln Cys Tyr Asn Cys Pro Asn Pro Thr Ala Asp Cys Lys Thr Ala
   1               5                   10                  15
   Val Ala Cys Ser Ser Asp Phe Asp Ala Cys Leu Ile Thr Lys Ala Gly
               20                  25                  30
   Leu Gln Val Tyr Asn Lys Cys Trp Lys Phe Glu His Cys Asn Phe Asn
           35                  40                  45
   Asp Val Thr Thr Arg Leu Arg Glu Asn Glu Leu Thr Tyr Tyr Cys Cys
       50                  55                  60
   Lys Lys Asp Leu Cys Asn
   65                  70
<210> 4
   <211> 82
   <212> PRT
   <213> Artificial Sequence
<220>
   <223> Synthetic polypeptide
<400> 4
   Leu Gln Cys Tyr Asn Cys Pro Asn Pro Thr Ala Asp Cys Lys Thr Ala
   1               5                   10                  15
   Val Asn Cys Ser Ser Asp Phe Asp Ala Cys Leu Ile Thr Lys Ala Gly
               20                  25                  30
   Leu Gln Val Tyr Asn Lys Cys Trp Lys Phe Glu His Cys Asn Phe Asn
           35                  40                  45
   Asp Val Thr Thr Arg Leu Arg Glu Asn Glu Leu Thr Tyr Tyr Cys Cys
       50                  55                  60
   Lys Lys Asp Leu Cys Asn Phe Asn Glu Gln Leu Glu Asn Gly Gly Thr
   65                  70                  75                  80
   Ser Cys
<210> 5
   <211> 83
   <212> PRT
   <213> Artificial Sequence
<220>
   <223> Synthetic polypeptide
<400> 5
   Met Leu Gln Cys Tyr Asn Cys Pro Asn Pro Thr Ala Asp Cys Lys Thr
   1               5                   10                  15
   Ala Val Asn Cys Ser Ser Asp Phe Asp Ala Cys Leu Ile Thr Lys Ala
               20                  25                  30
   Gly Leu Gln Val Tyr Asn Lys Cys Trp Lys Phe Glu His Cys Asn Phe
           35                  40                  45
   Asn Asp Val Thr Thr Arg Leu Arg Glu Asn Glu Leu Thr Tyr Tyr Cys
       50                  55                  60
   Cys Lys Lys Asp Leu Cys Asn Phe Asn Glu Gln Leu Glu Asn Gly Gly
   65                  70                  75                  80
   Thr Ser Cys
<210> 6
   <211> 71
   <212> PRT
   <213> Artificial Sequence
<220>
   <223> Synthetic polypeptide
<400> 6
   Leu Gln Cys Tyr Asn Cys Pro Asn Pro Thr Ala Asp Cys Lys Thr Ala
   1 5 10 15
   Val Ala Cys Ser Ser Asp Phe Asp Ala Cys Leu Ile Thr Lys Ala Gly
   20 25 30
   Leu Gln Val Tyr Asn Lys Cys Trp Lys Phe Glu His Cys Asn Phe Asn
   35 40 45
   Asp Val Thr Thr Arg Leu Arg Glu Asn Glu Leu Thr Tyr Tyr Cys Cys
   50 55 60
   Lys Lys Asp Leu Cys Asn Cys
   65 70
<210> 7
   <211> 99
   <212> PRT
   <213> Artificial Sequence
<220>
   <223> Synthetic polypeptide
<220>
   <221> DISULFID
   <222> (82)..(83)
   <223>
<400> 7
   Leu Gln Cys Tyr Asn Cys Pro Asn Pro Thr Ala Asp Cys Lys Thr Ala
   1 5 10 15
   Val Asn Cys Ser Ser Asp Phe Asp Ala Cys Leu Ile Thr Lys Ala Gly
   20 25 30
   Leu Gln Val Tyr Asn Lys Cys Trp Lys Phe Glu His Cys Asn Phe Asn
   35 40 45
   Asp Val Thr Thr Arg Leu Arg Glu Asn Glu Leu Thr Tyr Tyr Cys Cys
   50 55 60
   Lys Lys Asp Leu Cys Asn Phe Asn Glu Gln Leu Glu Asn Gly Gly Thr
   65 70 75 80
   Ser Cys Cys Asp Gly Pro Lys Lys Lys Lys Lys Lys Ser Pro Ser Lys
   85 90 95
   Ser Ser Gly
<210> 8
   <211> 100
   <212> PRT
   <213> Artificial Sequence
<220>
   <223> Synthetic polypeptide
<220>
   <221> DISULFID
   <222> (83)..(84)
   <223>
<400> 8
<210> 9
   <211> 88
   <212> PRT
   <213> Artificial Sequence
<220>
   <223> Synthetic polypeptide
<220>
   <221> DISULFID
   <222> (71)..(72)
   <223>
<400> 9
   Leu Gln Cys Tyr Asn Cys Pro Asn Pro Thr Ala Asp Cys Lys Thr Ala
   1 5 10 15
   Val Ala Cys Ser Ser Asp Phe Asp Ala Cys Leu Ile Thr Lys Ala Gly
   20 25 30
   Leu Gln Val Tyr Asn Lys Cys Trp Lys Phe Glu His Cys Asn Phe Asn
   35 40 45
   Asp Val Thr Thr Arg Leu Arg Glu Asn Glu Leu Thr Tyr Tyr Cys Cys
   50 55 60
   Lys Lys Asp Leu Cys Asn Cys Cys Asp Gly Pro Lys Lys Lys Lys Lys
   65 70 75 80
   Lys Ser Pro Ser Lys Ser Ser Gly
   85
<210> 10
   <211> 254
   <212> PRT
   <213> Artificial Sequence
<220>
   <223> Recombinant polypeptide
<400> 10
   Met Gln Asp Cys Gly Leu Pro Pro Asp Val Pro Asn Ala Gln Pro Ala
   1               5                   10                  15
   Leu Glu Gly Arg Thr Ser Phe Pro Glu Asp Thr Val Ile Thr Tyr Lys
               20                  25                  30
   Cys Glu Glu Ser Phe Val Lys Ile Pro Gly Glu Lys Asp Ser Val Ile
           35                  40                  45
   Cys Leu Lys Gly Ser Gln Trp Ser Asp Ile Glu Glu Phe Cys Asn Arg
       50                  55                  60
   Ser Cys Glu Val Pro Thr Arg Leu Asn Ser Ala Ser Leu Lys Gln Pro
   65                  70                  75                  80
   Tyr Ile Thr Gln Asn Tyr Phe Pro Val Gly Thr Val Val Glu Tyr Glu
                   85                  90                  95
   Cys Arg Pro Gly Tyr Arg Arg Glu Pro Ser Leu Ser Pro Lys Leu Thr
               100                 105                 110
   Cys Leu Gln Asn Leu Lys Trp Ser Thr Ala Val Glu Phe Cys Lys Lys
           115                 120                 125
   Lys Ser Cys Pro Asn Pro Gly Glu Ile Arg Asn Gly Gln Ile Asp Val
       130                 135                 140
   Pro Gly Gly Ile Leu Phe Gly Ala Thr Ile Ser Phe Ser Cys Asn Thr
   145                 150                 155                 160
   Gly Tyr Lys Leu Phe Gly Ser Thr Ser Ser Phe Cys Leu Ile Ser Gly
                   165                 170                 175
   Ser Ser Val Gln Trp Ser Asp Pro Leu Pro Glu Cys Arg Glu Ile Tyr
               180                 185                 190
   Cys Pro Ala Pro Pro Gln Ile Asp Asn Gly Ile Ile Gln Gly Glu Arg
           195                 200                 205
   Asp His Tyr Gly Tyr Arg Gln Ser Val Thr Tyr Ala Cys Asn Lys Gly
       210                 215                 220
   Phe Thr Met Ile Gly Glu His Ser Ile Tyr Cys Thr Val Asn Asn Asp
   225 230 235 240
   Glu Gly Glu Trp Ser Gly Pro Pro Pro Glu Cys Arg Gly Cys
   245 250
<210> 11
   <211> 271
   <212> PRT
   <213> Artificial Sequence
<220>
   <223> Synthetic polypeptide
<220>
   <221> DISULFID
   <222> (254)..(255)
   <223>
<220>
   <221> SITE
   <222> (271)..(271)
   <223> NH-(Myristoyl) attached to Gly271
<400> 11
   Met Gln Asp Cys Gly Leu Pro Pro Asp Val Pro Asn Ala Gln Pro Ala
   1 5 10 15
   Leu Glu Gly Arg Thr Ser Phe Pro Glu Asp Thr Val Ile Thr Tyr Lys
   20 25 30
   Cys Glu Glu Ser Phe Val Lys Ile Pro Gly Glu Lys Asp Ser Val Ile
   35 40 45
   Cys Leu Lys Gly Ser Gln Trp Ser Asp Ile Glu Glu Phe Cys Asn Arg
   50 55 60
   Ser Cys Glu Val Pro Thr Arg Leu Asn Ser Ala Ser Leu Lys Gln Pro
   65 70 75 80
   Tyr Ile Thr Gln Asn Tyr Phe Pro Val Gly Thr Val Val Glu Tyr Glu
   85 90 95
   Cys Arg Pro Gly Tyr Arg Arg Glu Pro Ser Leu Ser Pro Lys Leu Thr
   100 105 110
   Cys Leu Gln Asn Leu Lys Trp Ser Thr Ala Val Glu Phe Cys Lys Lys
           115                 120                 125
   Lys Ser Cys Pro Asn Pro Gly Glu Ile Arg Asn Gly Gln Ile Asp Val
       130                 135                 140
   Pro Gly Gly Ile Leu Phe Gly Ala Thr Ile Ser Phe Ser Cys Asn Thr
   145                 150                 155                 160
   Gly Tyr Lys Leu Phe Gly Ser Thr Ser Ser Phe Cys Leu Ile Ser Gly
                   165                 170                 175
   Ser Ser Val Gln Trp Ser Asp Pro Leu Pro Glu Cys Arg Glu Ile Tyr
               180                 185                 190
   Cys Pro Ala Pro Pro Gln Ile Asp Asn Gly Ile Ile Gln Gly Glu Arg
           195                 200                 205
   Asp His Tyr Gly Tyr Arg Gln Ser Val Thr Tyr Ala Cys Asn Lys Gly
       210                 215                 220
   Phe Thr Met Ile Gly Glu His Ser Ile Tyr Cys Thr Val Asn Asn Asp
   225                 230                 235                 240
   Glu Gly Glu Trp Ser Gly Pro Pro Pro Glu Cys Arg Gly Cys Cys Asp
                   245                 250                 255
   Gly Pro Lys Lys Lys Lys Lys Lys Ser Pro Ser Lys Ser Ser Gly
               260                 265                 270
<210> 12
   <211> 17
   <212> PRT
   <213> Artificial Sequence
<220>
   <223> Synthetic polypeptide
<220>
   <221> SITE
   <222> (1)..(1)
   <223> N-(myristoyl) attached to Gly1
<220>
   <221> SITE
   <222> (17)..(17)
   <223> NH2 attached to Cys17
<400> 12
   Gly Ser Ser Lys Ser Pro Ser Lys Lys Lys Lys Lys Lys Pro Gly Asp
   1 5 10 15
   Cys
<210> 13
   <211> 17
   <212> PRT
   <213> Artificial Sequence
<220>
   <223> Synthetic polypeptide
<220>
   <221> SITE
   <222> (1)..(1)
   <223> N-(myristoyl) attached to Gly1
<220>
   <221> SITE
   <222> (17)..(17)
   <223> (S-5-succinimido fluorescein)-NH2 attached to Cys17
<400> 13
   Gly Ser Ser Lys Ser Pro Ser Lys Lys Lys Lys Lys Lys Pro Gly Asp
   1 5 10 15
   Cys
<210> 14
   <211> 17
   <212> PRT
   <213> Artificial Sequence
<220>
   <223> Synthetic polypeptide
<220>
   <221> SITE
   <222> (1)..(1)
   <223> N-(myristoyl) attached to Gly1
<220>
   <221> SITE
   <222> (17)..(17)
   <223> (S--succinimido AlexafluorTM488C5)-NH2 attached to Cys17
<400> 14
   Gly Ser Ser Lys Ser Pro Ser Lys Lys Lys Lys Lys Lys Pro Gly Asp
   1               5                   10                  15
   Cys
<210> 15
   <211> 17
   <212> PRT
   <213> Artificial Sequence
<220>
   <223> Synthetic polypeptide
<220>
   <221> SITE
   <222> (1)..(1)
   <223> N-(myristoyl) attached to Gly1
<220>
   <221> SITE
   <222> (17)..(17)
   <223> (S--succinimido AlexafluorTM546 C5)-NH2 attached to Cys17
<400> 15
   Gly Ser Ser Lys Ser Pro Ser Lys Lys Lys Lys Lys Lys Pro Gly Asp
   1               5                   10                  15
   Cys

## Claims

1. A compound selected from the group consisting of
N-Myristoyl-Gly-Ser-Ser-Lys-Ser-Pro-Ser-Lys-Lys-Lys-Lys-Lys-Lys-Pro-Gly-Glu-Cys(S-5-succinimido fluorescein)-NH₂ (APT2087),
N-Myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (APT2104),
N-Myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (APT2105).

## Patentansprüche

1. Verbindung, ausgewählt aus der Gruppe bestehend aus N-Myristoyl-Gly-Ser-Ser-Lys-Ser-Pro-Ser-Lys-Lys-Lys-Lys-Lys-Lys-Pro-Gly-Glu-Cys(S-5-succinimidofluorescein)-NH₂ (APT2087),
N-Myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (APT2104),
N-Myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (APT2105).

## Revendications

1. Composé sélectionné parmi le groupe consistant en :
N-myristoyl-Gly-Ser-Ser-Lys-Ser-Pro-Ser-Lys-Lys-Lys-Lys-Lys-Lys-Pro-Gly-Glu-Cys(S-5-succinimido-fluorescéine)-NH₂ (APT2087),
N-myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (ATP2104),
N-myristoyl-Gly-Ser-Ser-Lys-Ser-Pro-Ser-Lys-Lys-Lys-Lys-Lys-Lys-Pro-Gly-Glu-Cys(S-5-succinimido Alexafluor^{™} 488C₅)-NH₂ (ATP2105).
